(19) Europäisches Patentamt
European Patent Office
Office européen des brevets

(11) Publication number: **0 111 583**
**B1**

(12) # EUROPEAN PATENT SPECIFICATION

(45) Date of publication of patent specification: **10.06.87**

(51) Int. Cl.⁴: **C 12 M 1/04**

(21) Application number: **82111853.6**

(22) Date of filing: **21.12.82**

(54) **Culture of anaerobic bacteria.**

(43) Date of publication of application:
**27.06.84 Bulletin 84/26**

(45) Publication of the grant of the patent:
**10.06.87 Bulletin 87/24**

(84) Designated Contracting States:
**DE FR GB**

(58) References cited:
**GB-A-2 083 496**

**CHEMICAL ABSTRACTS, vol. 92, no. 1, January 1980, page 460, no. 4728r, Columbus, Ohio, USA**

(73) Proprietor: **Toppan Printing Co., Ltd.**
**5-1, 1-chome, Taito**
**Taito-ku Tokyo (JP)**

(72) Inventor: **Omote, Kiyotaka**
**2-35-9-105, Aoba-cho**
**Higashimurayama-shi Tokyo (JP)**
Inventor: **Nakamura, Hachiro**
**405-72, Ooazanishikananoi Showa-machi**
**Kitakatsushika-gun Saitama-ken (JP)**

(74) Representative: **Bunke, Holger, Dr.rer.nat. Dipl.-Chem. et al**
**Patentanwälte Prinz, Leiser, Bunke & Partner**
**Manzingerweg 7**
**D-8000 München 60 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 111 583 B1

Courier Press, Leamington Spa, England.

## Description

The present invention relates to a method for culturing an anaerobic bacterium.

Anaerobic bacteria cannot utilize molecular oxygen because they do not have a cytochrome enzyme system, so they do not grow under usual culturing procedures. Since they grow only under conditions where no oxygen is present, culture media are required to be maintained under completely oxygen free conditions for the growth of anaerobic bacteria. To this end, various expedients are required and special devices and apparatuses are needed. Furthermore, operation of these devices and apparatuses requires a considerable degree of skill.

In culturing anaerobic bacteria, several procedures are known for removing molecular or free oxygen, as described below:

(a) Superposition method. This method is the most simple and involves superposing Vaseline® liquid paraffin, agar or the like on the surface of a medium to prevent the penetration of air.

(b) Deep layer solid medium culture method. This method involves boiling and then rapidly cooling a glucose-agar medium, a normal agar medium or the like, and subjecting an anaerobic bacterium to mixed culture or to stab culture with such a medium. This method utilizes the phenomenon that oxygen cannot penetrate into the depths of such a medium.

(c) Air substitution method. This method comprises culturing an anaerobic bacterium by placing a medium inoculated with a culture of the bacterium in a container made of iron or in a thick-walled glass container such as a desiccator, removing air from the container with a vacuum pump, and substituting hydrogen or nitrogen gas. Hosoya's culture device is a device which is relatively well-used in this method. This method is physically reliable, but various devices such as a vacuum pump, a gas cylinder and a pressure gauge are necessary, and laborious operations are also required.

(d) Chemical oxygen absorption method. This method achieves the removal of oxygen by oxygen absorption by means of a chemical reaction so as to maintain a closed system under anaerobic conditions. This method is named according to the chemicals used; the Schoetensack method and Rosenthal's chromate-sulfate method.

(i) Schoetensack method. This method comprises absorbing oxygen by reacting a mixture of gallic acid and sodium carbonate.

(ii) Rosenthal's chromate-sulfate method. This method involves absorbing oxygen by the generation of hydrogen due to the reaction of chromium metal with sulfuric acid.

$$Cr+H_2SO_4 \rightarrow CrSO_4+H_2$$

$$4CrSO_4+2H_2SO_4+O_2 \rightarrow 2Cr(SO_4)_3+2H_2O$$

(e) Method of Adding a Reducing agent or the like to Medium. This method involves rendering a culture medium anaerobic by the addition of a reducing agent or the like thereto. The media to be used for this method may include chopped liver bouillon or Hosoya Cysteine Bouillon.

(f) Biological Method. This method involves culturing anaerobic bacteria in a culture medium which is rendered anaerobic by decreasing the molecular oxygen in a container by utilizing the aspiration of aerobic bacteria. This method requires a mycelium mass of Serratia marcescens or Bacillus subtilis which is well cultured in advance.

Although each of the methods illustrated above has its own merits, they are all laborious methods of rendering culture conditions anaerobic. It has recently been established that carbon dioxide gas is necessary to the culture of anaerobic bacteria, and culturing is now generally carried out in the presence of carbon dioxide and in the absence of oxygen.

GB—A—2 083 496 discloses a method of cultivating anaerobic bacterium by using an oxygen-removing composition containing fillers such as ferrous carbonate, an oxide or hydroxide of an alkali metal or alkaline earth metal, and activated carbon. It is taught that the oxygen concentration and carbon dioxide concentration of the cultivating atmosphere should be made 0.1% or less and 5 to 15%, respectively, within 10 hours.

The present invention has as its object to provide a method for culturing an anaerobic bacterium, that is, creating an anaerobic atmosphere where concentrations of oxygen and carbon dioxide are within predetermined ranges within a short period of time. This innovation will enable public health centers, general/clinics and hospitals to culture anaerobic bacteria. Heretofore, the culture of anaerobic bacteria was possible only in large hospitals or medical institutes which were equipped with bacterial inspection facilities.

Furthermore, the object of the present invention is to provide a process for culturing anaerobic bacteria which comprises removing molecular oxygen in a closed system by the absorption of such oxygen by means of a chemical reaction, simultaneously generating carbon dioxide to a concentration dependent upon the type of anaerobic bacteria, and then maintaining such an atmosphere for a predetermined period of time for culturing said anaerobic bacteria.

A further object of the present invention is to provide the culturing of anaerobic bacteria in a favorable manner in an anaerobic culture atmosphere of a closed system which is generated within 6 hours to a point

where the oxygen concentration is 0.1% or lower and the carbon dioxide concentration ranges from 5 to 50%.

The present invention provides a method for culturing an anaerobic bacterium comprising placing a composition for generating an anaerobic atmosphere in a gas permeable bag made of a gas permeable sheet; separately placing the gas permeable bag and a culture medium inoculated with the anaerobic bacterium in an envelope; and hermetically closing the envelope and culturing the anaerobic bacterium under an anaerobic atmosphere which is formed as a result of a chemical reaction of the composition for generating an anaerobic atmosphere comprising not more than 0.1% oxygen and 5 to 50% carbon dioxide; wherein said composition comprises ascorbic acid or salt thereof as an oxygen-absorbing agent; a hydrated carbonate of an alkali metal or an alkaline earth metal; a bicarbonate of an alkali metal; and a metal compound selected from ferrous compounds, and cuprous or cupric compounds.

According to another aspect of the present invention, there is provided a method for culturing an anaerobic bacterium, comprising placing a composition for generating an anaerobic atmosphere in a gas permeable bag made of a gas permeable sheet; separately placing the gas permeable bag and a culture medium inoculated with the anaerobic bacterium in an envelope; and hermetically closing the envelope and culturing the anaerobic bacterium under an anaerobic atmosphere which is formed as a result of a chemical reaction of the composition for generating an anaerobic atmosphere comprising not more than 0.1% oxygen and 5 to 50% carbon dioxide; wherein said composition comprises a hydrated ferrous salt as an oxygen-absorbing agent, two kinds of alkaline compounds, one of which is a hydroxide of an alkali metal or alkaline earth metal, and the other a carbonate, and a sulfite of an alkaline earth metal.

If the invention uses as an oxygen absorbing agent a hydrated ferrous salt such as hydrated ferrous sulfate or hydrated ferrous chloride as a major ingredient, two further different kinds of alkaline compounds, such as hydroxide or carbonate of alkali metal or alkaline earth metal are used together with said oxygen absorbing agent. Varying ratios of such a carbonate and a hydroxide may be used to adjust the concentrations of oxygen and carbon dioxide.

The reasons for using two different kinds of alkaline compounds are as follows. The use of alkali or alkaline earth metal hydroxide (such as $Ca(OH_2)$), which has a high reactivity with ferrous sulfate, is required to facilitate the oxygen absorption of hydrated ferrous salt (such as $FeSO_4 \cdot 7H_2O$) as the major component and of calcium sulfite. As will be shown in the reaction schemes below, water liberated in Reaction (1) (preferably water liberated in the initial reaction of ferrous sulfate heptahydrate) is subjected to the absorption of water and generation of carbon dioxide by the ferrous sulfate and one of the alkaline compounds, i.e., a carbonate such as calcium carbonate according to Reaction (2) and to the oxygen absorption reaction by means of alkaline earth metal sulfite such as calcium sulfite according to Reaction (3).

(1) $$2FeSO_4 \cdot 7H_2O+2Ca(OH)_2+H_2O+1/2O_2 \rightarrow 2Fe(OH)_2+2CaSO_4 \cdot 2H_2O+10H_2O$$

(2) $$2FeSO_4 \cdot 7H_2O+2CaCO_3+3H_2O+1/\ 2O_2 \rightarrow 2Fe(OH)_3+2CaSO_4 \cdot 2H_2O+2CO_2+10H_2O$$

(3) $$CaSO_3 \cdot 1/2H_2O+1/2O_2+3/2H_2O \rightarrow CaSO_4 \cdot 2H_2O$$

It is accordingly found that the composition used according to the present invention requires two different kinds of alkaline compounds such as hydroxides of alkali metal or alkaline earth metal and a carbonate in order to cause the initial reaction as described above and to cause the reaction for generating carbon dioxide to occur.

The amount of carbon dioxide to be generated may be adjusted by varying the amount of a carbonate which functions as a source for carbon dioxide generation.

The hydrated ferrous salts to be used according to the present invention may include, for example, $FeSO_4 \cdot 5H_2O$, $FeSO_4 \cdot 7H_2O$, $FeCl_2 \cdot 4H_2O$. Ferrous sulfate heptahydrate is most preferred.

The alkaline earth metal sulfite may include, for example, $CaSO_3 \cdot 1/2H_2O$, $CaSO_3 \cdot 2H_2O$, $BaSO_3$. Among them, $CaSO_3 \cdot 1/2H_2O$ is most preferred.

The hydroxides of alkali metal and alkaline earth metal may include, for example, KOH, NaOH, LiOH, $Ca(OH)_2$, $Ba(OH)_2$, $Mg(OH)_2$.

Carbonates of alkali metal or alkaline earth metal includes $K_2CO_3$, $Na_2CO_3$, $Li_2CO_3$, $CaCO_3$, $BaCO_3$, $MgCO_3$, $BeCO_3$, $KHCO_3$, $NaHCO_3$, $LiHCO_3$ or their hydrates such as $Na_2CO_3 \cdot 10H_2O$, $MgCO_3 \cdot 3H_2O$, $Na_2CO_3 \cdot 7H_2O$, $BeCO_3 \cdot 4H_2O$, $KNaCO_3 \cdot 6H_2O$.

The porous carrier may include, for example, activated carbon, zeolite, bentonite, activated clay and activated alumina, with activated carbon being most preferred.

The mixing ratios of each of the ingredients may range as follows.

| | |
|---|---|
| Hydrated ferrous salt | 4.0 (weight) |
| Sulfite of alkaline earth metal | 1.0 (weight) |
| Hydroxides of alkali metal or alkaline earth metal | 0.5 (weight) |
| Carbonates of alkali metal or alkaline earth metal | 0.25 to 2 (weight) |

In order to control the concentration of carbon dioxide in the range of 5 to 50% in the atmosphere, the ratio of carbonate in relative to hydroxide should be adjusted in the range of 0.5:1 to 4:1. 0.5 part by weight of the porous carrier may be included in the above composition.

Table 1 which follows illustrates examples of blending each of the ingredients based on the amounts of carbon dioxide generated. In the table below, units are in parts by weight.

TABLE 1

| Ingredient | Amount of carbon dioxide gas generated | | |
|---|---|---|---|
| | 5—10% | 10—30% | 30—50% |
| Ferrous sulfate heptahydrate | 4.0 | 4.0 | 4.0 |
| Calcium hydroxide | 0.5 | 0.5 | 0.5 |
| Calcium sulfite semihydrate | 1.0 | 1.0 | 1.0 |
| Calcium carbonate | 0.25—0.5 | 0.5—1.5 | 1.5—2.5 |
| Activated carbon | 0.5 | 1.0 | 0.5 |

If the present invention uses as an oxygen absorbing agent ascorbic acid or salts thereof such as sodium L-ascorbate as a major ingredient, two different kinds of alkaline compounds, namely, hydrated alkali metal or alkaline earth metal carbonates such as $Na_2CO_3 \cdot 10\ H_2O$ and a bicarbonate or alkali metal such as $NaHCO_3$ are used together with said oxygen absorbing agent. The concentrations of oxygen and carbon dioxide may be adjusted by varying the mixing ratios of the major ingredient and the alkaline compounds. The use of the two different kinds of alkaline compounds as illustrated above is for the following reasons. In order to facilitate and speed-up the reaction when ascorbic acid or its salt is the major component, water and a catalyst are required. Although the catalyst to be used here may be ferrous sulfate (for the initial reaction) and activated carbon, various techniques for supplying the water may be considered. For example, there may be included a step in which water is added at the time of filling or blending and a process in which hydrated active carbon is employed. These processes, however, are accompanied by practical difficulties in that the water is not dispersed homogeneously, or the reaction proceeds too quickly.

In accordance with the present invention, as a technique capable of dispersing water uniformly and homogeneously, a hydrated carbonate of alkali or alkaline earth metal as an alkaline compound is chosen as a prerequisite ingredient in order to maintain a pH balance in a system and to supply water. Since this alkaline compound has the property to liberate about 90% of its hydrate at temperatures higher than about 35°C, it can supply water uniformly during the step of blending or granulating.

The use of such ingredients according to the latter aspect of the present invention causes ascorbic acid or its salt, for example, sodium L-ascorbate to proceed with the oxygen absorption reaction as shown in Reaction Step (4) below. The other alkaline compound, i.e., a bicarbonate, functions as a source for carbon dioxide gas generation. A main reaction involved in this step is shown in a reaction scheme below. For example, oxalic acid is reacted with sodium bicarbonate according to Reaction (5) below.

(4)

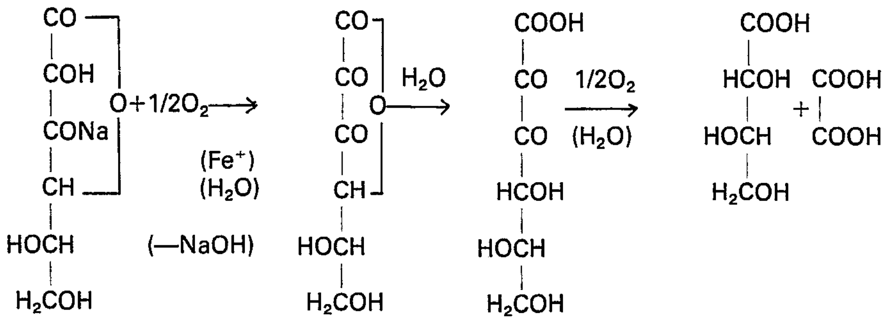


(5)

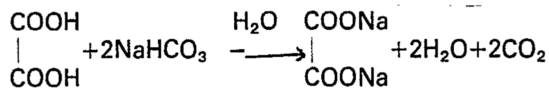


Accordingly, the composition used in accordance with this aspect of the present invention requires two different kinds of alkaline compounds, i.e., a hydrated carbonate of alkali metal or alkaline earth metal and a bicarbonate of alkali metal, in order to provide a desired anaerobic atmosphere.

Adjustment of the amounts of carbon dioxide generated may be achieved by varying the amounts of the hydrated carbonate and the bicarbonate used.

An ascorbic acid and salts thereof (ascorbate) to be used in accordance with the present invention may include, for example, L-ascorbic acid, calcium L-ascorbate, sodium L-ascorbate, D-iso-ascorbic acid and sodium D-iso-ascorbate. The most preferred is sodium L-ascorbate.

The hydrated carbonate of alkali metal or alkaline earth metal may include, for example, $Na_2CO_3 \cdot 7H_2O$, $Na_2CO_3 \cdot 10H_2O$, $BeCO_3 \cdot 4H_2O$, $KNaCO_3 \cdot 6H_2O$ and $MgCO_3 \cdot 6H_2O$. Among them, $Na_2CO_3 \cdot 10H_2O$ is most preferred.

The bicarbonate of alkali metal may include, for example, $KHCO_3$, $NaHCO_3$ and $LiHCO_3$. Sodium bicarbonate is generally preferred.

The ferrous compound and cuprous or cupric compound may include, for example, $FeSO_4$, $FeSO_4 \cdot 7H_2O$, $FeSO_4 \cdot 10H_2O$, $FeCl_2$, $FeCl_2 \cdot 4H_2O$, $CuSO_4$, $CuSO_4 \cdot 5H_2O$, $CuCl$, $CuCl_2$, $CuCl_2 \cdot 2H_2O$.

As the porous carrier may be used, for example, activated carbon, zeolite, bentonite, activated clay and activated alumina. Activated carbon is generally preferred.

The mixing ratios of each of the ingredients may range as follows.

| | |
|---|---|
| Ascorbic acid and its salt | 6 (weight) |
| Hydrated carbonate of alkali metal or alkaline earth metal | 1 to 12 (weight) |
| Bicarbonate of alkali metal | 1 to 12 (weight) |
| Ferrous compounds, cuprous or cupric compound | 1 or more (weight) |
| Porous carrier | 5 or more (weight) |

In order to control the concentration, in the atmosphere, of carbon dioxide in the range of 5 to 50%, the ratio of the alkaline compounds in relative to ascorbic acid or its salt should be adjusted in the range of 0.3:1 to 4:1. The above two kinds of alkaline compounds should be added in equal amounts.

Table 2 which follows illustrates examples of blends based on the amount of carbon dioxide generated. In Table 2 below, units are in parts by weight.

TABLE 2

| Ingredient | Amount of carbon dioxide gas generated | | |
|---|---|---|---|
| | 5—10% | 10—30% | 30—50% |
| Sodium L-ascorbate | 6 | 6 | 6 |
| Sodium carbonate decahydrate | 1—3 | 3—10 | 8—12 |
| Sodium bicarbonate | 1—3 | 3—6 | 6—12 |
| Ferrous sulfate heptahydrate | 1 | 1 | 1 |
| Activated carbon | 5 | 5 | 5 |

The amount of the compositions as illustrated hereinabove may be varied according to the amount of air remaining in a closed system, and with the time required for the removal of oxygen by absorption.

The compositions used according to the present invention may be employed by being packed with a suitable gas permeable packing material.

The compositions may be stored by being packed with a packing material incapable of ventilating oxygen in order to prevent any oxygen-absorbing reaction from occurring during storage.

The compositions used in accordance with the present invention may merely be placed in a container such as a laboratory dish or a Kolben culture test tube which is inoculated with anaerobic bacteria to be cultured, or in a jar in which a variety of test specimens are placed, or in a synthetic resin bag having a low gas permeability to gases such as oxygen, carbon dioxide, vapor or the like. Accordingly, this process may render an atmosphere anaerobic in a very simple manner.

The favorable aspects to be accomplished by the present invention lies in the fact that agents A and B to be used in accordance with the present invention both release and absorb little heat at the start of a reaction with air, and that a reaction can start within a wide range of temperatures. This process accordingly is applicable to mesophiles and thermophiles as well as to psychrophiles. Furthermore, this process does not disperse or absorb water during the reaction with air, so that a culture medium contained in a closed system neither gets too dry nor too moist.

The present invention will be described in more detail by way of working examples and with reference to the drawing which is a cross-sectional view illustrating a process for culturing anaerobic bacteria according to the present invention.

Example 1

A deoxidizer having the composition illustrated below was placed in a gas permeable bag 2 filled with 600 $cm^3$ of air and made of a paper of fine quality which was laminated with polyethylene film equipped in its inner layer with small pores. This bag was then placed in a bag 4 made of a laminate body consisting of a 50 µm thick polyethylene film and a 20 µm thick polypropylene film coated with vinylidene chloride. Inside the bag 4 was also placed a laboratory dish 1 containing an agar gum culture inoculated with *B. melaninogenicus SS. intermedius* (hereinafter referred to as bacterium A) and absorbent cotton 3 containing Fildes-Mcintosh indicator as an anaerobic indicator and sealed in a manner indicated in the drawing. The culture was carried out at 35 to 37°C for 48 hours.

TABLE 3

| | A—1 | A—2 | A—3 | A—4 |
|---|---|---|---|---|
| Ferrous sulfate heptahydrate | 8.0 | 8.0 | 8.0 | 8.0 |
| Calcium hydroxide | 1.2 | 1.0 | 1.0 | 1.0 |
| Calcium sulfite semihydrate | 2.0 | 2.0 | 2.0 | 2.0 |
| Calcium carbonate | 1.5 | 0.5 | 3.0 | 5.5 |
| Activated carbon | 1.0 | 1.0 | 1.0 | 1.0 |
| | Comparative Example | Present invention | Present invention | Comparative Example |

| | B—1 | B—2 | B—3 | B—4 |
|---|---|---|---|---|
| Sodium L-ascorbate | 1.2 | 1.2 | 1.2 | 1.2 |
| Sodium carbonate decahydrate | 0.2 | 0.2 | 2.4 | 1.0 |
| Sodium bicarbonate | 0.1 | 0.2 | 2.4 | 3.0 |
| Ferrous sulfate heptahydrate | 0.2 | 0.2 | 0.2 | 0.2 |
| Activated carbon | 1.0 | 1.0 | 1.0 | 1.0 |
| | Comparative Example | Present Invention | Present Invention | Comparative Example |

Table 4 below illustrates the concentrations of gases ($O_2/CO_2$) in the bag 4 measured after 6 hours.

TABLE 4
Test results (gas concentrations in container after culturing for 6 hours at 35—37°C)

| | A—1 | A—2 | A—3 | A—4 | B—1 | B—2 | B—3 | B—4 |
|---|---|---|---|---|---|---|---|---|
| $O_2$ | 1.0 | —<0.1 | <0.1 | 1.5 | 0.8 | <0.1 | <0.1 | 1.2 |
| $CO_2$ | 2.5 | 5.1 | 49.5 | 60.3 | 3.5 | 5.0 | 49.7 | 57.7 |

As shown above, A—1, A—4, B—1 and B—4 are beyond the scope defined in accordance with the present invention and do not satisfy the requirements for 0.1% or less of $O_2$ and 5 to 50% of $CO_2$ within 6 hours. In the tests, the absorbent cotton 3 containing the indicator turned white. Furthermore, bacteria propagated to the extent that they could be observed from outside the bag. Observation after opening the bag was accordingly unnecessary, unlike in conventional procedures.

Example 2

A deoxidizer containing the same composition as in Example 1 (A—1 to A—4 and B—1 to B—4) was placed in a bag consisting of a laminate body made of a polyethylene film and a polypropylene film coated with vinylidene chloride together with a laboratory dish containing an agar gum medium (manufactured by Nisui Seiyaku K.K.) inoculated with *B. melaninogenicus SS. intermedius* and *B. melaninogenicus SS. nedaninogenicus*. The bag was sealed and then cultured at 37°C for 48 hours. Table 5 below indicates results of the culture.

TABLE 5

| | | A—1 | A—2 | A—3 | A—4 | B—1 | B—2 | B—3 | B—4 |
|---|---|---|---|---|---|---|---|---|---|
| B. melaminogenicus ss. *intermedicus* | | – | – | + | – | – | – | + | – |
| B. melaminogenicus ss. *medaninogenicus* | | – | + | + | – | – | + | + | – |
| Gas concentrations after culturing for 6 hours at 37°C | $O_2$ | 1.0 | 0.1 | 0.1 | 1.5 | 0.8 | 0.1 | 0.1 | 1.2 |
| | $CO_2$ | 2.5 | 5.1 | 49.5 | 60.3 | 3.5 | 5.0 | 49.7 | 57.7 |

As shown in the results above, excellent propagation of bacteria was observed in A—2, A—4, B—2 and B—3 which each comprised a composition according to the present invention.

**Claims**

1. A method for culturing an anaerobic bacterium, comprising placing a composition for generating an anaerobic atmosphere in a gas permeable bag made of a gas permeable sheet; separately placing the gas permeable bag and a culture medium inoculated with the anaerobic bacterium in an envelope; and hermetically closing the envelope and culturing the anaerobic bacterium under an anaerobic atmosphere which is formed as a result of a chemical reaction of the composition for generating an anaerobic atmosphere comprising not more than 0.1% oxygen and 5 to 50% carbon dioxide; wherein said composition comprises ascorbic acid or salt thereof as an oxygen-absorbing agent; a hydrated carbonate of an alkali metal or an alkaline earth metal; a bicarbonate of an alkali metal; and a metal compound selected from ferrous compounds, and cuprous or cupric compounds.

2. A method for culturing an anaerobic bacterium, comprising placing a composition for generating an anaerobic atmosphere in a gas permeable bag made of a gas permeable sheet; separately placing the gas permeable bag and a culture medium inoculated with the anaerobic bacterium in an envelope; and hermetically closing the envelope and culturing the anaerobic bacterium under an anaerobic atmosphere which is formed as a result of a chemical reaction of the composition for generating an anaerobic atmosphere comprising not more than 0.1% oxygen and 5 to 50% carbon dioxide; wherein said composition comprises a hydrated ferrous salt as an oxygen-absorbing agent, two kinds of alkaline compounds, one of which is a hydroxide of an alkali metal or alkaline earth metal, and the other a carbonate, and a sulfite of an alkaline earth metal.

3. A method according to claim 1, wherein the total amount of said hydrated carbonate and bicarbonate is 0.3 to 4 parts by weight relative to 1 part by weight of said oxygen-absorbing agent.

4. A method according to claim 1, wherein the composition for generating an anaerobic atmosphere comprises 6 parts by weight of sodium L-ascorbate, 1 to 12 parts by weight of sodium carbonate decahydrate, 1 to 12 parts by weight of sodium bicarbonate, 1 part by weight of ferrous sulfate heptahydrate, and 5 parts by weight of activated carbon.

5. A method according to claim 2, wherein a weight ratio of said carbonate to said hydroxide is 0.5:1 to 4:1.

6. A method according to claim 2, wherein the composition for generating an anaerobic atmosphere comprises 4.0 parts by weight of ferrous sulfate heptahydrate, 0.5 parts by weight of calcium hydroxide, 1.0 part by weight of calcium sulfite semihydrate, and 0.25 to 2.5 parts by weight of calcium carbonate.

**Patentansprüche**

1. Verfahren zum Kultivieren eines anaeroben Bakteriums, bei dem eine Zusammensetzung zur Erzeugung einer anaeroben Atmosphäre in einen gasdurchlässigen Beutel aus einer gasdurchlässigen Folie gebracht wird, der gasdurchlässige Beutel und ein mit dem anaeroben Bakterium beimpftes Kulturmedium getrennt voneinander in eine Hülle gestellt werden, die Hülle hermetisch verschlossen und das anaerobe Bakterium unter einer anaeroben Atmosphäre kultiviert wird, die als Ergebnis einer chemischen Reaktion der Zusammensetzung zur Erzeugung einer anaeroben Atmosphäre mit nicht mehr als 0,1% Sauerstoff und 5 bis 50% Kohlendioxid gebildet wird, wobei die Zusammensetzung Ascorbinsäure oder eines ihrer Salze als Sauerstoff absorbierendes Mittel, ein hydratisiertes Carbonat eines Alkali- oder Erdalkalimetalls, ein Bicarbonat eines Alkalimetalls sowie eine Metallverbindung umfaßt, die aus den Eisen(II)-, Kupfer(I)- oder Kupfer(II)-Verbindungen ausgewählt ist.

2. Verfahren zum Kultivieren eines anaeroben Bakteriums, bei dem eine Zusammensetzung zur Erzeugung einer anaeroben Atmosphäre in einen gasdurchlässigen Beutel aus einer gasdurchlässigen Folie gebracht wird, der gasdurchlässige Beutel und ein mit dem anaeroben Bakterium beimpftes Kulturmedium getrennt voneinander in eine Hülle gestellt werden, die Hülle hermetische verschlossen und das anaerobe Bakterium unter einer anaeroben Atmosphäre kultiviert wird, die als Ergebnis einer chemischen Reaktion der Zusammensetzung zur Erzeugung einer anaeroben Atmosphäre mit nicht mehr als 0,1% Sauerstoff und 5 bis 50% Kohlendioxid gebildet wird, wobei die Zusammensetzung ein hydratisiertes Eisen(II)-Salz als Sauerstoff absorbierendes Mittel, zwei Arten von alkalischen Verbindungen, von denen die eine ein Hydroxid eines Alkali- oder Erdalkalimetalls und die andere ein Carbonat ist, sowie ein Sulfit eines Erdalkalimetalls umfaßt.

3. Verfahren nach Anspruch 1, bei dem die Gesamtmenge des hydratisierten Carbonats und Bicarbonats 0,3 bis 4 Gewichtsteile, bezogen auf ein Gewichtsteil des Sauerstoff absorbierenden Mittels, beträgt.

4. Verfahren nach Anspruch 1, bei dem die Zusammensetzung zur Erzeugung einer anaeroben Atmosphäre 6 Gewichtsteile Natrium-L-ascorbat, 1 bis 12 Gewichtsteile Natriumcarbonat-Decahydrat, 1 bis 12 Gewichtsteile Natriumcarbonat, 1 Gewichtsteil Eisen(II)sulfat-Heptahydrat sowie 5 Gewichtsteile Aktivkohle umfaßt.

5. Verfahren nach Anspruch 2, bei dem das Gewichtsverhältnis zwischen dem Carbonat und dem Hydroxid 0,5:1 bis 4:1 beträgt.

6. Verfahren nach Anspruch 2, bei dem die Zusammensetzung zur Erzeugung einer anaeroben Atmosphäre 4,0 Gewichtsteile Eisen(II)sulfat-Heptahydrat, 0,5 Gewichtsteile Calciumhydroxid, 1,0 Gewichtsteile Calciumsulfit-Semihydrat und 0,25 bis 2,5 Gewichtsteile Calciumcarbonat umfaßt.

**Revendications**

1. Procédé pour la culture d'une bactérie anaérobie, comprenant l'introduction d'une composition pour la production d'une atmosphère anaérobie dans un sac perméable aux gaz, fait d'une feuille perméable aux gaz; la disposition, séparément, du sac perméable aux gaz et d'un milieu de culture ensemencé avec la bactérie anaérobie dans une enveloppe; et la fermeture hermétique de l'enveloppe et la culture de la bactérie anaérobie dans une atmosphère anaérobie qui est formée à la suite d'une réaction chimique de la composition pour la production d'une atmosphère anaérobie comprenant 0,1% d'oxygène au maximum et de 5 à 50% d'anhydride carbonique, dans lequel procédé ladite composition comprend de l'acide ascorbique ou un sel de celui-ci en tant qu'agent absorbant l'oxygène, un carbonate hydraté d'un métal alcalin ou d'un métal alcalino-terreux, un bicarbonate d'un métal alcalin, et un composé métallique choisi parmi des composés ferreux et des composés cuivreux ou cuivriques.

2. Procédé pour la culture d'une bactérie anaérobie, comprenant l'introduction d'une composition pour la production d'une atmosphère anaérobie dans un sac perméable aux gaz, fait d'une feuille perméable aux gaz; la disposition, séparément, du sac perméable aux gaz et d'un milieu de culture ensemencé avec la bactérie anaérobie dans une enveloppe; et la fermeture hermétique de l'enveloppe et la culture de la bactérie anaérobie dans une atmosphère anaérobie qui est formée à la suite d'un réaction chimique de la composition pour la production d'une atmosphère anaérobie comprenant 0,1% d'oxygène au maximum et de 5 à 50% d'anhydride carbonique, dans lequel procédé ladite composition comprend un sel ferreux hydraté en tant qu'agent absorbant l'oxygène, deux sortes de composés alcalins, dont l'un est un hydroxyde d'un métal alcalin ou d'un métal alcalino-terreux et l'autre est un carbonate, et un sulfite d'un métal alcalino-terreux.

3. Procédé selon la revendication 1, dans lequel la quantité totale dudit carbonate hydraté et dudit bicarbonate va de 0,3 à 4 parties en poids par rapport à 1 partie en poids dudit agent absorbant l'oxygène.

4. Procédé selon la revendication 1, dans lequel la composition pour la production d'une atmosphère anaérobie comprend 6 parties en poids de L-ascorbate de sodium, 1 à 12 parties en poids de carbonate de sodium décahydraté, 1 à 12 parties en poids de bicarbonate de sodium, 1 partie en poids de sulfate ferreux heptahydraté, et 5 parties en poids de charbon actif.

5. Procédé selon la revendication 2, dans lequel le rapport pondéral dudit carbonate audit hydroxyde va de 0,5:1 à 4:1.

6. Procédé selon la revendication 2, dans lequel la composition pour la production d'une atmosphère anaérobie comprend 4,0 parties en poids de sulfate ferreux heptahydraté, 0,5 partie en poids d'hydroxyde de calcium, 1,0 partie en poids de sulfite de calcium hémihydraté et 0,25 à 2,5 parties en poids de carbonate de calcium.

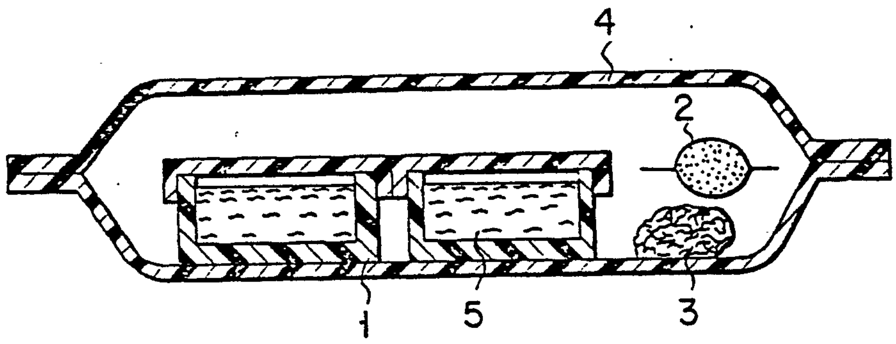
4
2
1
5
3